# EUROPÄISCHE PATENTSCHRIFT

(11) **EP 0 930 862 B1**
(45) Veröffentlichungstag und Bekanntmachung des Hinweises auf die Patenterteilung: **17.07.2002**
(21) Anmeldenummer: 97910319.9
(22) Anmeldetag: 19.09.1997
(51) Int. Cl.: A61F 13/15

(54) **ABSORBIERENDER ARTIKEL**
ABSORBENT ARTICLE
ARTICLE ABSORBANT

(30) Priorität: 30.09.1996 DE 19640451
(43) Veröffentlichungstag der Anmeldung: 28.07.1999
(73) Patentinhaber: Hakle-Kimberly Deutschland GmbH, 56070 Koblenz-Rheinhafen (DE)
(72) Erfinder: RAIDEL, Maria, D-90451 Nürnberg (DE); ASCHENBRENNER, Franz, D-92280 Kastl (DE)
(74) Vertreter: Diehl, Hermann, Dr. Dipl.-Phys.
(86) Internationale Anmeldenummer: EP9705160
(87) Internationale Veröffentlichungsnummer: WO9814151

(56) Entgegenhaltungen:
- EP-A- 0 119 919
- EP-A- 0 343 941
- EP-A- 0 570 016
- WO-A-97/33546
- DE-U- 9 218 991

## Beschreibung

Die vorliegende Erfindung betrifft einen absorbierenden Artikel.

Absorbierende Artikel sind als Hygieneprodukte seit langem bekannt. Sie finden beispielsweise als Windeln, Inkontinenzeinlagen oder Damenbinden Verwendung. Diese absorbierenden Artikel sind so ausgelegt, daß sie flüssige Körperausscheidungen, wie Urin, Menstruationsflüssigkeit oder Blut, aufnehmen und speichern können. Damenbinden werden beispielsweise eingesetzt, um die vor, während und nach der Menstruation ausgeschiedenen Flüssigkeiten zu absorbieren. Damenbinden werden außen (extern) am Körper getragen und unterscheiden sich insofern von Tampons, welche in die weibliche Vagina eingeführt werden und somit als "interne" Produkte bezeichnet werden können.

Als nachteilig wird beim Gebrauch von bekannten absorbierenden Artikeln häufig empfunden, daß die dem Körper zugewandte Oberfläche nach Beaufschlagung mit einer Flüssigkeit nasse Bereiche aufweist, was beim Träger zu einem unangenehmen Gefühl führt. Untersuchungen in diesem Zusammenhang haben ergeben, daß beispielsweise herkömmliche Damenbinden bereits rücknässen, wenn erst ca. 5% des theoretischen Flüssigkeitsaufnahmevermögens der Binde ausgeschöpft sind. Desweiteren hinterlassen die ausgeschiedenen Körperflüssigkeiten auf der Oberfläche des verwendeten absorbierenden Artikels häufig sichtbare Rückstände, was den Verwender des Artikels dazu verleitet, den absorbierenden Artikel häufiger auszutauschen als dies von der Aufnahmefähigkeit für Flüssigkeiten her notwendig wäre.

EP-0119919-A1 offenbart eine Damenbinde, deren Form an die weibliche Körperanatomie angepaßt ist und die einen schichtförmigen Aufbau aufweist. Die Damenbinde umfaßt eine völlig flüssigkeitsundurchlässige Lage, über der verschiedene absorbierende Schichten angeordnet sind. Über dieser flüssigkeitsundurchlässigen Lage ist als obere Schicht auf der dem Körper der Trägerin zugewandten Seite eine Lage aus Vliesstoff angebracht, die ebenfalls eine ovale Öffnung aufweisen kann.

Der vorliegenden Erfindung liegt somit die Aufgabe zugrunde, einen absorbierenden Artikel zur Verfügung zu stellen, bei dem die Aufnahmefähigkeit des Flüssigkeitsspeichermaterials des absorbierenden Artikels optimal genützt wird und wobei auch nach längerem Gebrauch des absorbierenden Artikels auf der dem Körper zugewandten Seite möglichst wenig Gebrauchsspuren sichtbar sind.

Diese Aufgabe löst die vorliegende Erfindung durch den absorbierenden Artikel gemäß dem unabhängigen Patentanspruch 1. Weitere vorteilhafte Ausgestaltungen des absorbierenden Artikels ergeben sich aus den abhängigen Patentansprüchen, der Beschreibung und der Zeichnung.

Der erfindungsgemäße absorbierende Artikel weist folgenden Aufbau auf. Auf der beim Gebrauch des absorbierenden Artikels dem Körper abgewandten Seite befindet sich eine flüssigkeitsundurchlässige Schicht. Oberhalb dieser flüssigkeitsundurchlässigen Schicht ist eine Primärspeicherschicht angeordnet. Daraufhin schließt sich nach oben eine Sekundärspeicherschicht an. Oberhalb der Sekundärspeicherschicht ist eine Kompensationsschicht angeordnet, und oberhalb der Kompensationsschicht befindet sich eine Deckschicht, welche eine zentrale Öffnung aufweist, und wobei die Deckschicht eine Mischung aus Zellstoff und polymerisiertem Alken enthält, in der die Mischung mindestens 50 Gew.-% polymerisiertes Alken enthält.

Schließlich enthält der absorbierende Artikel gemäß der vorliegenden Erfindung noch eine obere, bei Gebrauch des absorbierenden Artikels dem Körper zugewandten flüssigkeitsdurchlässige Schicht. Es hat sich als vorteilhaft erwiesen, wenn die Sekundärspeicherschicht zumindest einen verdichteten Bereich aufweist. Eine besonders günstige Speicherung in dem erfindungsgemäßen absorbierenden Artikel wird erreicht, wenn der zumindest eine verdichtete Bereich in Längsrichtung des Artikels verläuft. Dabei sollte der zumindest eine in Längsrichtung des Artikels verlaufende verdichtete Bereich zumindest innerhalb der Projektion der zentralen Öffnung der Deckschicht auf die Sekundärspeicherschicht verlaufen. Besonders günstige Ergebnisse werden erzielt, wenn die Sekundärspeicherschicht zumindest fünf verdichtete Bereiche aufweist. Als Material für die Sekundärspeicherschicht ist beispielsweise Zellstoff geeignet. Lokale Verdichtungen in dem Sekundärspeicher können beispielsweise durch Einprägen von Rillen in den Speicher erfolgen. Das unterhalb der eingeprägten Rillen gelegene Speichermaterial ist dabei verdichtet, während die Rillen zur gerichteten Flüssigkeitsverteilung auf der Speicherschicht bzw. in dem absorbierenden Artikel beitragen.

Die Deckschicht, welche die zentrale Öffnung aufweist, wird aus einer Mischung aus Zellstoff und polymerisiertem Alken hergestellt. Entsprechende Mischungen enthalten mindestens 50 Gew.-% polymerisiertes Alken. Sehr gute Ergebnisse werden erzielt, wenn der Anteil an polymerisiertem Alken 50-80 Gew.-%, insbesondere 60 Gew.-% beträgt. Die Deckschicht kann auch aus zwei Schichten aufgebaut sein derart, daß eine erste Schicht aus einem Gemisch aus Zellstoff und polymerisiertem Alken auf einer zweiten Trägerschicht aus polymerisiertem Alken aufgebracht ist, wobei die erste Schicht aus einem Gemisch aus Zellstoff und polymerisiertem Alken mit der bei Gebrauch des absorbierenden Artikels dem Körper zugewandten flüssigkeitsdurchlässigen Schicht und die zweite Trägerschicht mit der Kompensationsschicht in Verbindung steht. Bevorzugte polymerisierte Alkene sind Polyethylen, Polypropylen und Gemische aus Polyethylen und Polypropylen. Die Deckschicht kann des weiteren ein Pigment, wie Titandioxid, enthalten. Das Material der Kompensationsschicht ist vorteilhafterweise aus einem Vliesmaterial aufgebaut. Das Vliesmaterial kann polymerisiertes Alken und/oder Bikomponentenfasern enthalten. Auch kann die Kompensationsschicht auf der der Speicherschicht zugewandten Oberfläche mit einer oberflächenaktiven Substanz, welche beispielsweise siliconhaltig sein kann, beschichtet sein. Die Primärspeicherschicht kann beispielsweise aus einem UCTAD-Material, Tissuewatte oder einem polymeren Alken bestehen. Die Primärspeicherschicht ist vorteilhafterweise so aufgebaut, daß deren Randbereiche derart eingefaltet sind, daß diese sich gegenseitig überlappen.

Sowohl die flüssigkeitsundurchlässige Schicht als auch die flüssigkeitsdurchlässige Schicht können aus einem polymerisierten Alken, wie beispielsweise Polyethylen, Polypropylen oder einem Gemisch daraus aufgebaut sein. Zur Befestigung des absorbierenden Artikels an einem Kleidungsstück ist an der flüssigkeitsundurchlässigen Schicht vorteilhafterweise mindestens ein Haftelement und/oder eine Haftschicht angebracht. Desweiteren kann der erfindungsgemäße absorbierende Artikel auch seitlich angeordnete Flügel aufweisen. Der erfindungsgemäße absorbierende Artikel findet bevorzugt als Hygieneprodukt, insbesondere als Damenbinde oder Damen-Hygieneeinlage Verwendung.

Ein bevorzugtes UCTAD-Material (uncreped through air dried-Material), wie es auch als Primärspeicherschicht Verwendung finden kann, enthält mindestens 10 Gew.-% hochergiebige Zellstofffasern (high yield pulp fibers), bezogen auf die Trockensubstanz, zu denen ein Naßfestmittel in einer Menge zugegeben wird, die bewirkt, daß das Verhältnis der Naßreißfestigkeit zu der Trockenreißfestigkeit mindestens etwa 0,1 beträgt. Hochergiebige Zellstofffasern enthalten viel Lignin, auf welche die Naßelastizität der Fasern zurückzuführen sein dürfte. Die durch das Naßfestmittel ausgebildeten Harzbindungen immobilisieren die naßelastischen Fasern in einer blattartigen Struktur, welche sich der Struktur des Bandes anpaßt, auf dem die Trocknung (throughdrying) erfolgt. Während des Trocknungsvorganges werden die von dem Naßfestmittel gebildeten Bindungen ausgehärtet, so daß sich nässeresistente Bindungen ausbilden können, was wiederum die hochelastischen Eigenschaften einer entsprechenden Bahn im nassen Zustand bewirkt. Diese Eigenschaft behält die Bahn bei, da bei einem UCTAD-Verfahren kein Kreppschritt oder anderer Schritt, welcher die Bindungen wieder zerstören könnte, durchgeführt wird. Somit ist das UCTAD-Material hervorragend geeignet, Flüssigkeiten zu transportieren und zu speichern, da das Material auch im nassen Zustand stabilisiert ist. Nachfolgend sollen einige Vorteile des erfindungsgemäßen absorbierenden Artikels näher erläutert werden.

Die oberste, dem Körper zugewandte Schicht, welche flüssigkeitsdurchlässig ist, kann beispielsweise aus spinngebundenem Polypropylen hergestellt sein. Wenn diese flüssigkeitsdurchlässige obere Abdeckschicht mit geeigneten Pigmenten, wie beispielsweise Titandioxid, versetzt ist, verhindert diese Schicht bis zu einem gewissen Ausmaß ein Durchscheinen der in dem absorbierenden Artikel gespeicherten Flüssigkeit. Diese oberste, dem Körper zugewandte Schicht kann- auch ein Vlies- oder Folienmaterial mit einer zentral angeordneten Öffnung sein.

Als nächst tiefere Schicht folgt die Deckschicht, welche zum leichteren Eindringen der ausgeschiedenen Flüssigkeiten in darunterliegende Schichten eine zentrale Öffnung aufweist. Diese Öffnung kann beispielsweise ausgestanzt sein und eine ovale oder eine hundeknochenförmige Kontur aufweisen. Auch die Deckschicht kann mit Pigmenten versehen sein, was wiederum bewirkt, daß auf der Oberfläche des absorbierenden Artikels bei Beaufschlagung eingedrungene Flüssigkeit nicht sichtbar wird. Eine weitere Funktion der Deckschicht ist es, ein Zurückschlagen eingedrungener Flüssigkeit auf die Oberfläche zu verhindern, so daß keine Rücknässung auftritt und der Körper des Trägers trockengehalten wird. Dies wird dadurch erreicht, daß die Deckschicht aus einem Coformmaterial aufgebaut ist. Ein geeignetes Coformmaterial ist beispielsweise eine Polypropylen-Zellstoff-Mischung mit einem Polypropylengehalt von mehr als 50 Gew.-%. Polypropylengehalte von 50-80 Gew.-% und insbesondere von 60 Gew.-% haben sich als besonders günstig erwiesen. Coformmaterial wird gewonnen, indem Zellstoff zerfasert und schmelzgeblasenes Polypropylen hergestellt wird. Der zerfaserte Zellstoff und die Polypropylenfasern werden dann gemischt und auf einem sich bewegenden Band abgelegt, wodurch die gewünschten Polypropylen-Zellstoff-Mischungen erhalten werden. Durch den Aufbau des Coformmaterials, d.h. durch dessen Zellstoffanteil, saugt diese Deckschicht von oben kommende Flüssigkeit auf und gibt sie nach unten ab. Dies ist insbesondere dann wichtig, wenn zum Beispiel eine Damenbinde falsch plaziert ist, mit die Flüssigkeit nicht über die ausgestanzte Öffnung direkt über die Folie in die Binde eintritt. Der mehr als 50%-ige Polypropylenanteil des Coformmaterials dagegen bewirkt, daß die Deckschicht von unten einmal in die Binde eingedrungene Flüssigkeit nicht mehr aufnimmt. Das Coformmaterial bewirkt somit, daß die Rücknässeigenschaften des absorbierenden Artikels erheblich verbessert werden, wobei bei einer entsprechenden Pigmentierung auch ein Durchscheinen aufgenommener Flüssigkeit verhindert wird (sogenannter stain-hiding-Effekt).

Die Rücknässeigenschaften des erfindungsgemäßen absorbierenden Artikels können weiter verbessert werden, wenn die Deckschicht zwei- oder dreilagig aufgebaut ist. Die obere, dem Körper zugewandte Schicht, kann dabei das eben beschriebene Coformmaterial, welches eine Mischung aus Polypropylen und Zellstoff ist, sein. Dieses Coformmaterial ist dann vorzugsweise auf eine weitere Schicht aus polymerisiertem Alken, wie beispielsweise Polypropylen aufgebracht. Auch dreilagige Strukturen mit einer zwischen zwei Schichten aus polymerisiertem Alken sandwichartig angeordneten Coformschicht sind möglich. Das Polypropylen kann über ein Spinnbindeverfahren hergestellt sein, wodurch sich eine vliesartige Struktur ergibt. Die Hydrophobizität des polymeren Alkens bewirkt, daß einmal in dem Saugkörper befindliche Flüssigkeit von einer Rückkehr an die Oberfläche des absorbierenden Artikels wirksam abgehalten wird.

Die Kompensationsschicht (Surge-Schicht) ist vorzugsweise aus einem Vliesmaterial aufgebaut. Das Vlies kann aus Polyethylen, Polypropylen oder anderen polymerisierten Alkenen bestehen sowie Bikomponentenfasern enthalten. Falls es sich bei dem absorbierenden Artikel um eine Windel handelt, hat diese Kompensationsschicht sowohl Speicher- als auch Verteilfunktion für den aufgenommenen Urin, insbesondere als Zwischenspeicher. Handelt es sich bei dem absorbierenden Artikel dagegen um eine Damenbinde, hat die Kompensationsschicht keine Speicherfunktion. Die Kompensationsschicht dient bei Damenbinden in erster Linie wieder dazu, eingedrungene Flüssigkeit unsichtbar zu halten. Neben Vliesmaterialien können für die Kompensationsschicht auch kardierte Bahnen verwendet werden. Besonders wirkungsvoll sind Kompensationsschichten, die mit einer oberflächenaktiven Substanz behandelt sind. Besonders geeignet sind siliconhaltige oberflächenaktive Mittel. Die oberflächenaktiven Mittel werden auf der Unterseite der Kompensationsschicht aufgebracht, d.h. auf der Seite, mit der diese auf dem Saugkörper (Sekundärspeicher) aufliegt. Durch die dadurch bewirkten Kapillaritätseffekte wird ein Rücknässen durch die Kompensationsschicht wirksam verhindert.

Eine besondere Funktion hat bei dem absorbierenden Artikel der Saugkörper. Er dient hierbei nicht allein der Speicherung, sondern auch der Verteilung der eingedrungenen Flüssigkeit, wobei der Speicher auch nur als Sekundärspeicher agiert, wie nachfolgend noch näher erläutert werden wird. Die Flüssigkeitsverteilfunktion des Saugkörpers kann durch eine Spezialprägung unterstützt werden. Dabei wird die Dichte des Saugkörpers in Längsrichtung des absorbierenden Artikels gesehen lokal erhöht. Die Verdichtung kann beispielsweise durch Einprägen von Linien oder Rillen erfolgen. Die Prägung sollte sich zumindest in dem Bereich befinden, welche unterhalb der zentralen Öffnung der Deckschicht gelegen ist. Die Prägung kann jedoch auch weiter bis in die Endbereiche des absorbierenden Körpers ausgedehnt sein. Ein bevorzugter absorbierender Körper enthält fünf in Längsrichtung angeordnete verdichtete Bereiche. Einprägungen bewirken, daß dort die Speicherfähigkeit des Saugkörpers reduziert ist, die eingedrungene Flüssigkeit in den Vertiefungen verteilt und an die darunterliegende Primärspeicherschicht abgegeben wird. Außerdem wird durch die Prägung im Mittelbereich des Saugkörpers dessen Dicke reduziert, was dazu führt, daß der absorbierende Artikel am Rand dicker ist als im mittleren Bereich. Durch diese Vertiefung im Mittelbereich erhöht sich der Tragekomfort beispielsweise einer Damenbinde. Wenn dann der Saugkörper mit Flüssigkeit beaufschlagt ist, besteht durch die Vertiefung im Mittelbereich weniger Körperkontakt der Binde mit der Haut des Trägers bzw. der Trägerin, wodurch sich das Trockenheitsgefühl auf der Körperoberfläche erhöht. Ein besonders geeignetes Material für den Saugkörper bzw. den Sekundärspeicher ist Zellstoff.

Die Primärspeicherschicht liegt direkt auf der flüssigkeitsundurchlässigen Schicht (Wäscheschutzfolie) auf. Sie besteht vorzugsweise aus einem UCTAD-Material, Tissuewatte oder einem schmelzgeblasenen Polypropylen. Die Primärspeicherschicht dient neben dem Speichern von Flüssigkeit auch der Verteilung eingedrungener Flüssigkeiten in die Endbereiche des absorbierenden Körpers. Die Verteilung in die Endbereiche kann noch unterstützt werden, wenn die Primärspeicherschicht im Mittelbereich des absorbierenden Artikels schmäler ist als in den Endbereichen. Wenn der Primärspeicher beispielsweise aus Tissuewatte aufgebaut ist, ist er in der Lage, bis ca. zwei Milliliter Flüssigkeit aufzunehmen. Untersuchungen haben aufgezeigt, daß mehr als die Hälfte aller verwendeten Damenbinden mit nicht mehr als zwei Milliliter Flüssigkeit beaufschlagt werden, bevor sie ausgetauscht werden. Dies bedeutet, daß bei einem Großteil gebrauchter Damenbinden die gesamte aufgenommene Flüssigkeit in der neben der Wäscheschutzfolie dem Körper entferntesten Schicht aufgenommen ist. Es liegt auf der Hand, daß dadurch eine Rücknässung optimal unterbunden und auch ein Durchscheinen der aufgenommenen Flüssigkeit auf der körperzugewandten Seite einer Damenbinde praktisch nicht auftreten wird. Sollte die Beaufschlagung zwei Milliliter übersteigen, so gibt die Primärspeicherschicht darüber hinaus mehr eingedrungene Flüssigkeit nach oben in die Sekundärspeicherschicht ab, wodurch ein sogenannter "bottom-up-filling-Effekt" auftritt, jedoch erst bei zwei Milliliter übersteigenden Flüssigkeitsmengen.

Der Primärspeicher kann auch aus einer gewellten bzw. plissierten Bahn bestehen, wobei durch die Wellung die Verteilfunktion der Schicht unterstützt wird. Die Wellen der Bahn sind dabei so angeordnet, daß die Flüssigkeit in Längsrichtung zu den Enden des Artikels hin abgeleitet wird. Dabei verlaufen die Wellen in Querrichtung des absorbierenden Artikels derart, daß die Wellen in Längsrichtung Förderkanäle bilden.

Die Erfindung wird nachfolgend anhand der Zeichnungen näher erläutert. Es zeigen:
- Fig. 1: eine Draufsicht auf einen erfindungsgemäßen absorbierenden Artikel mit ovaler zentraler Öffnung in der Deckschicht;
- Fig. 2: eine Draufsicht auf einen erfindungsgemäßen absorbierenden Artikel mit hundeknochenförmiger zentraler Öffnung in der Deckschicht;
- Fig. 3: einen Querschnitt durch den erfindungsgemäßen absorbierenden Artikel gemäß Fig. 1 entlang der Linie III-III;
- Fig. 4: eine Draufsicht auf einen erfindungsgemäßen absorbierenden Artikel mit Flügeln;
- Fig. 5: einen Querschnitt durch den erfindungsgemäßen absorbierenden Artikel gemäß Fig. 4 entlang der Linie IV-IV; sowie
- Fig. 6: eine perspektivische Ansicht einer Primärspeicherschicht in Form einer gewellten Bahn.

Die vorliegende Erfindung wird nachfolgend anhand der Figuren und bevorzugter Ausführungsformen, welche als Damenbinden ausgestaltet sind, näher erläutert.

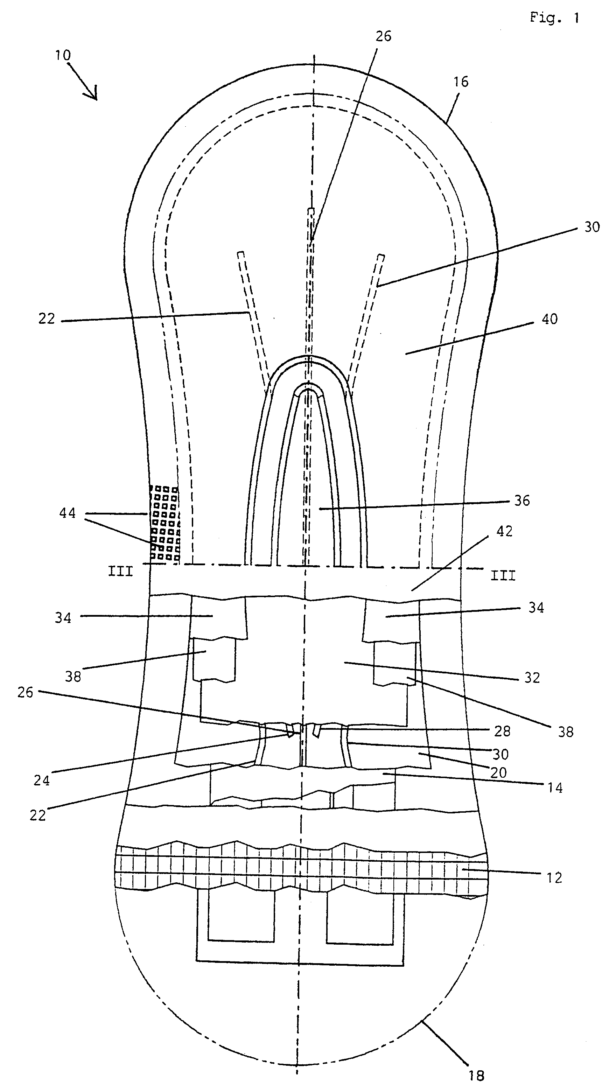

Fig. 1 zeigt die Draufsicht auf einen erfindungsgemäßen Artikel 10. Im unteren Bereich der Fig. 1 sind Lagen bzw. Schichten des erfindungsgemäßen Artikels 10 teilweise weggelassen, um darunterliegende Schichten sichtbar zu machen. Die unterste Schicht des Artikels 10 wird durch eine flüssigkeitsundurchlässige Schicht 12 gebildet, welche mit einem Rechteckmuster gekennzeichnet ist. Die flüssigkeitsundurchlässige Schicht 12 besteht aus einer Polypropylenfolie. Die flüssigkeitsundurchlässige Schicht 12 dient dabei als sogenannte Wäscheschutzfolie, welche verhindert, daß in den absorbierenden Artikel eingedrungene und dort zurückgehaltene Flüssigkeit nach unten aus dem absorbierenden Körper austreten kann. Dadurch wird verhindert, daß die Unterbekleidung des Trägers bzw. der Trägerin verschmutzt wird.

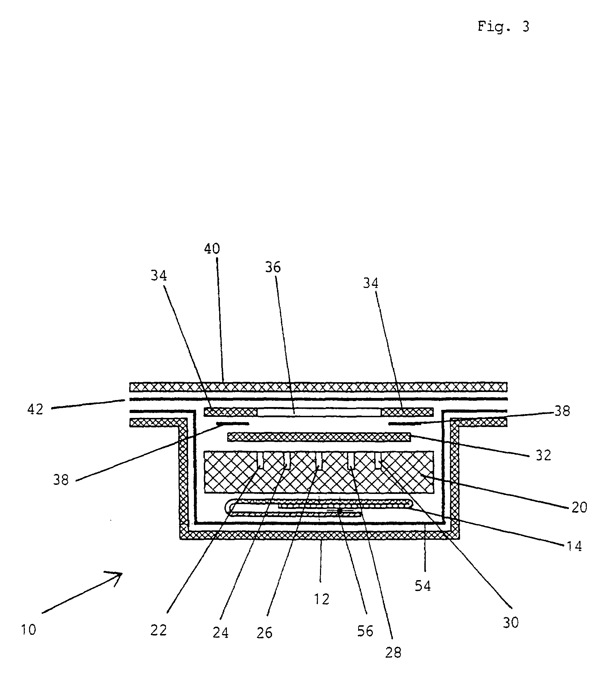

Auf der flüssigkeitsundurchlässigen Schicht 12 liegt die Primärspeicherschicht 14, welche aus einem Tissuewatte-Material besteht, auf. Die Längskanten der die Primärspeicherschicht 14 bildenden Bahnen sind derart eingeschlagen, daß diese sich unterhalb der Bahn überlappen, wie aus Fig. 3 näher ersichtlich wird. Die Primärspeicherschicht ist in der Lage, ca. zwei Milliliter Flüssigkeit aufzunehmen und zu speichern. Desweiteren dient die Primärspeicherschicht 14 dazu, die in den zentralen Bereich des absorbierenden Artikels 10 eingedrungene Flüssigkeit in den vorderen und hinteren Endbereich 16, 18 des absorbierenden Artikels zu verteilen.

Die nächstfolgende Schicht wird von dem Sekundärspeicher 20 gebildet, der aus einer Zellstofflage besteht. In die Zellstofflage sind Rillen 22, 24, 26, 28 und 30 eingeprägt. Auf dem Sekundärspeicher 20 auftreffende Flüssigkeit wird bevorzugt durch die Rillen 20, 24, 26, 28 und 30 in den vorderen und hinteren Bereich des absorbierenden Artikels 10 weitertransportiert und nach unten in die Primärspeicherschicht 14 abgegeben. Durch die Einprägung der Rillen 20, 24, 26, 28, 30 wird der Sekundärspeicher 20 lokal verdichtet, wodurch die Saugfähigkeit des Sekundärspeichers 20 lokal verringert wird, was wiederum eine Abgabe der eingedrungenen Flüssigkeit an die darunterliegende Primärspeicherschicht 14 erleichtert. Damit wird gewährleistet, daß vor einer Auffüllung des Sekundärspeichers 20 mit eingedrungener Flüssigkeit die Primärspeicherschicht 14 sich mit Flüssigkeit vollsaugen kann. Erst wenn die Kapazitätsgrenze der Primärspeicherschicht 14 erreicht ist, gibt diese wieder Flüssigkeit an die darüberliegende Sekundärspeicherschicht 20 ab, wodurch sich der sogenannte "bottom-up-filling-Effekt" ergibt.

Auf dem Sekundärspeicher 20 liegt die Kompensationsschicht 32 auf. Die Kompensationsschicht 32 besteht aus einem Vlies aus spinngebundenem Polypropylen. Sie dient vor allem dem schnellen Eindringen der Flüssigkeit in den absorbierenden Artikel und verhindert die Rücknässung sowie ein Durchscheinen der eingedrungenen Flüssigkeit an der Oberfläche des absorbierenden Artikels.

Oberhalb der Kompensationsschicht 32 ist die Deckschicht 34 angeordnet. Die Deckschicht 34 weist eine ovale, zentrale Öffnung 36 auf.

Die Deckschicht 34 ist mit der Kompensationsschicht 32 durch eine Heißschmelzkleberschicht 38 randseitig verbunden. Dadurch wird ein Verrutschen der beiden Schichten gegeneinander verhindert. Die Deckschicht besteht aus einem Coformmaterial aus Zellstoff und Polypropylen, wobei der Polypropylenanteil des Gemischs 60 Gew.-% beträgt. Desweiteren ist in die Deckschicht ein Titandioxidpigment eingelagert. Die zentrale, ovale Öffnung 36 wird während des Herstellungsverfahrens der Deckschicht ausgestanzt.

Die körperseitige Abdecklage des erfindungsgemäßen absorbierenden Artikels gemäß Fig. 1 wird durch die flüssigkeitsdurchlässige Schicht 40 aus einem spinngebundenen Polypropylenvliesmaterial gebildet, welches zusätzlich Titandioxid zur Pigmentierung enthält. Die flüssigkeitsdurchlässige Schicht 40 ist mit der Deckschicht 34 und im Bereich der zentralen Öffnung 36 der Deckschicht 34 mit der Kompensationsschicht 32 über eine Heißschmelzkleberschicht 42 verbunden, wodurch der Schichtenaufbau des absorbierenden Artikels stabilisiert wird. Im Außenrandbereich weist der absorbierende Artikel 10 noch eine Randprägung 44 auf.

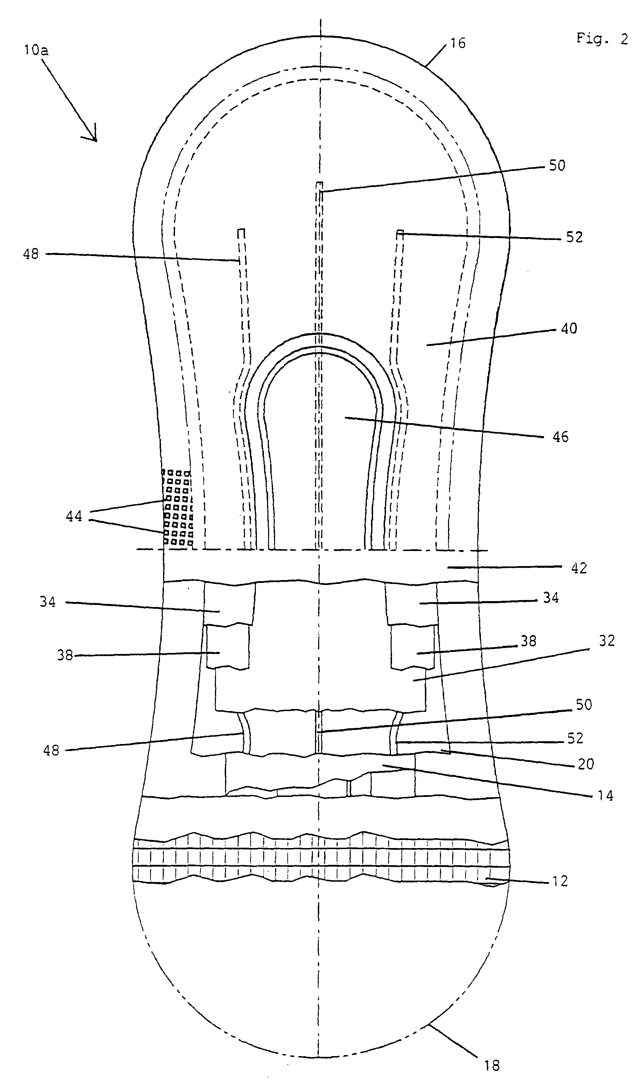

In Fig. 2 wird ein absorbierender Artikel 10a gezeigt, der in seinem wesentlichen Aufbau dem in Zusammenhang mit Fig. 1 beschriebenen absorbierenden Artikel 10 entspricht. Der absorbierende Artikel 10a unterscheidet sich von dem absorbierenden Artikel 10 in der Form der zentralen Öffnung der Deckschicht 34. Die zentrale Öffnung 46 der Deckschicht des absorbierenden Artikels 10a ist bei der Ausführungsform gemäß Fig. 2 hundeknochenförmig ausgestaltet. Desweiteren sind die geprägten Rillen 48, 50, 52 anders ausgestaltet als bei dem absorbierenden Artikel 10a. Beide Arten der Ausgestaltung der Rillen gewährleisten eine effektive Verteilung eingedrungener Flüssigkeiten in die Endbereiche (16, 18) der absorbierenden Artikel 10, 10a. Die übrigen Elemente des in Fig. 2 gezeigten absorbierenden Artikels 10a entsprechen den Elementen des in Figur 1 gezeigten absorbierenden Artikels 10 und sind dementsprechend mit denselben Bezugsziffern gekennzeichnet.

Fig. 3 stellt einen Querschnitt der in Fig. 1 gezeigten Damenbinde entlang der Linie III-III dar. Die Bezugsziffer 12 bezeichnet wiederum die flüssigkeitsundurchlässige Schicht, welche als Wäscheschutzfolie dient. Die flüssigkeitsundurchlässige Schicht 12 ist mit einer Heißschmelzkleberschicht 54 beschichtet, welche in Fig. 1 nicht sichtbar wird. Auf der Heißschmelzkleberschicht 54 ist die Primärspeicherschicht 14 aufgebracht. Die Primärspeicherschicht 14 ist randseitig eingeklappt, wobei sich die eingeklappten Enden überlappen. Die überlappenden Enden sind mittels einer Heißschmelzkleberlinie 56 miteinander verklebt. An die Primärspeicherschicht 14 schließt sich nach oben die Sekundärspeicherschicht 20 an. In die Sekundärspeicherschicht 20 sind die Rillen 22, 24, 26, 28 und 30 eingeprägt. Oberhalb der Sekundärspeicherschicht 20 befindet sich die Kompensationsschicht 32, welche mittels der Heißschmelzkleberschicht 38 mit der Deckschicht 34 in Verbindung steht. Die Deckschicht 34 weist die zentrale, ovale Öffnung 36 auf. Die obere Abdecklage des erfindungsgemäßen absorbierenden Artikels 10 bildet die flüssigkeitsdurchlässige Schicht 40, welche über die Heißschmelzkleberschicht 42 mit den darunterliegenden Schichten verbunden ist. Bei Gebrauch des erfindungsgemäßen absorbierenden Artikels werden die einzelnen Schichten aufeinandergedrückt, so daß diese jeweils unmittelbar miteinander in Verbindung stehen. Der besseren Übersichtlichkeit wegen wurden in Fig. 3 die einzelnen Schichten isoliert stehend gezeichnet.

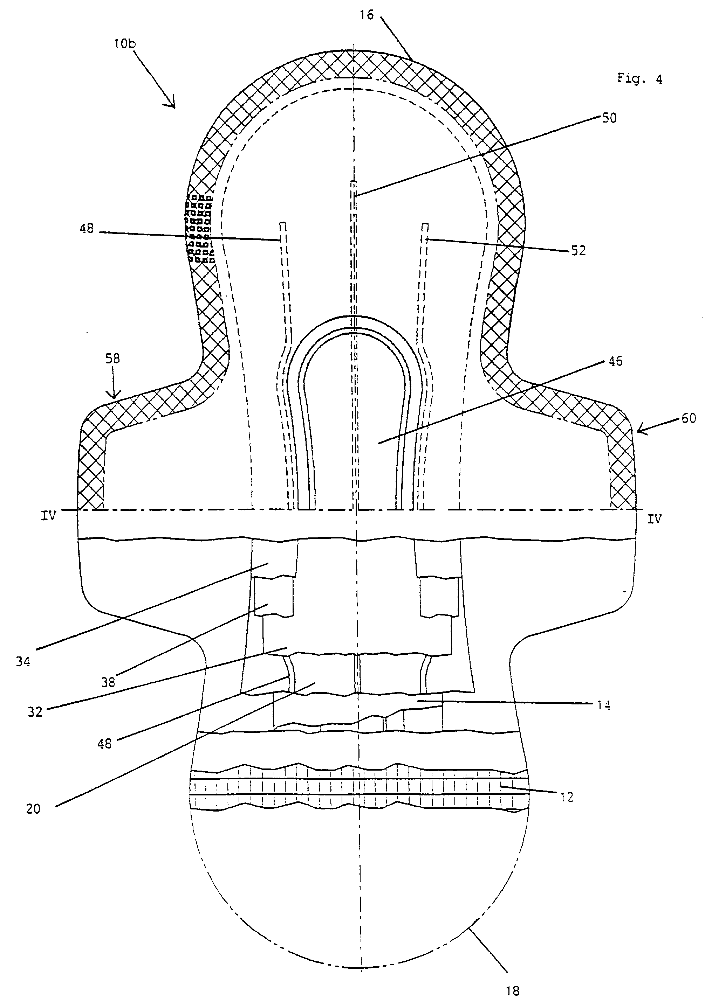

Fig. 4 zeigt eine weitere erfindungsgemäße Ausgestaltung des absorbierenden Artikels, welcher in wesentlichen Bereichen seines Aufbaus dem in Fig. 2 gezeigten absorbierenden Artikel 10a entspricht. Zur besseren Befestigung des absorbierenden Artikels 10b an der Kleidung einer Trägerin weist dieser randseitig gelegene Flügelbereiche 58, 60 auf. Bei Gebrauch des Artikels 10b werden die Flügelbereiche 58, 60 nach unten umgeklappt und auf der Außenseite der Unterbekleidung miteinander verbunden. Der übrige Aufbau und die übrigen Bezugsziffern in Fig. 4 entsprechen denen der Fig. 2.

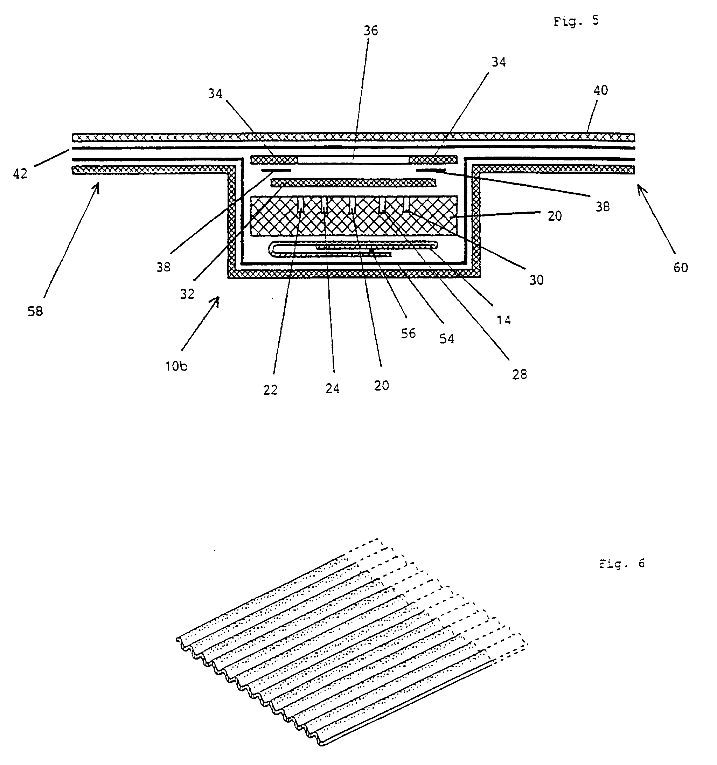

Fig. 5 zeigt einen Querschnitt des absorbierenden Artikels 10b aus Fig. 4 entlang der Linie IV-IV. Die Flügelbereiche 58, 60 sind in Fig. 5 ebenfalls deutlich erkennbar. Der übrige Aufbau und die übrigen Bezugsziffern aus Fig. 5 entsprechen denen aus Fig. 3.

Fig. 6 schließlich zeigt eine gewellte Bahn aus einem UCTAD-Material, welche als Primärspeicherschicht 14 in den absorbierenden Artikel 10, 10a, 10b gemäß der Erfindung verwendet werden kann. Durch die in Querrichtung zu dem absorbierenden Artikel verlaufende Wellung werden Förderkanäle gebildet, welche eine noch effektivere Flüssigkeitsverteilung des im zentralen Bereich beaufschlagten absorbierenden Artikels in die Endbereiche 16, 18 bewirkt.

## Patentansprüche

1. Absorbierender Artikel (10; 10a; 10b) mit folgendem Aufbau:
einer unteren, bei Gebrauch des absorbierenden Artikels dem Körper abgewandten, flüssigkeitsundurchlässigen Schicht (12),
einer oberhalb der flüssigkeitsundurchlässigen Schicht (12) angeordneten Primärspeicherschicht (14),
einer oberhalb der Primärspeicherschicht (14) angeordneten Sekundärspeicherschicht (20),
einer oberhalb der Sekundärspeicherschicht (20) angeordneten Kompensationsschicht (32),
einer oberhalb der Kompensationsschicht (32) angeordneten Deckschicht (34), welche eine zentrale Öffnung (36; 46) aufweist, und
einer oberen, bei Gebrauch des absorbierenden Artikels dem Körper zugewandten, flüssigkeitsdurchlässigen Schicht (40),
**dadurch gekennzeichnet, dass** die Deckschicht (34) eine Mischung aus Zellstoff und polymerisiertem Alken enthält und wobei die Mischung mindestens 50 Gew.-% polymerisiertes Alken enthält.

2. Absorbierender Artikel (10; 10a; 10b) nach Anspruch 1, wobei die Sekundärspeicherschicht (20) zumindest einen verdichteten Bereich aufweist.

3. Absorbierender Artikel (10; 10a; 10b) nach Anspruch 2, wobei der zumindest eine verdichtete Bereich in Längsrichtung des Artikels verläuft.

4. Absorbierender Artikel (10; 10a; 10b) nach Anspruch 3, wobei der zumindest eine in Längsrichtung des Artikels verlaufende verdichtete Bereich zumindest innerhalb der Projektion der zentralen Öffnung (36; 46) der Deckschicht (34) auf die Sekundärspeicherschicht (20) angeordnet ist.

5. Absorbierender Artikel (10; 10a; 10b) nach einem der Ansprüche 2 bis 4, wobei die Sekundärspeicherschicht (20) fünf verdichtete Bereiche aufweist.

6. Absorbierender Artikel (10; 10a; 10b) nach einem der vorhergehenden Ansprüche, wobei die Sekundärspeicherschicht (20) aus Zellstoff aufgebaut ist.

7. Absorbierender Artikel (10; 10a; 10b) nach einem der vorhergehenden Ansprüche, wobei der Anteil an polymerisierten Alken 50 - 80 Gew.-%, insbesondere 60 Gew.-% enthält.

8. Absorbierender Artikel (10; 10a; 10b) nach einem der vorhergehenden Ansprüche, wobei die Deckschicht (34) aus zwei Schichten aufgebaut ist, derart, daß eine erste Schicht aus einem Gemisch aus Zellstoff und polymerisiertem Alken auf einer zweiten Trägerschicht aus polymerisiertem Alken aufgebracht ist, wobei die erste Schicht aus einem Gemisch aus Zellstoff und polymerisiertem Alken mit der bei Gebrauch des absorbierenden Artikels dem Körper zugewandten flüssigkeitsdurchlässigen Schicht (40) und die zweite Trägerschicht mit der Kompensationsschicht (32) in Verbindung steht.

9. Absorbierender Artikel (10; 10a; 10b) nach einem der Ansprüche 1 bis 7, wobei die Deckschicht (34) aus zwei Schichten aufgebaut ist, derart, daß eine erste Schicht aus einem Gemisch aus Zellstoff und polymerisiertem Alken auf einer zweiten Trägerschicht aus polymerisiertem Alken aufgebracht ist, wobei die erste Schicht aus einem Gemisch aus Zellstoff und polymerisiertem Alken mit der Kompensationsschicht (32) und die zweite Trägerschicht mit der bei Gebrauch des absorbierenden Artikels dem Körper zugewandten flüssigkeitsdurchlässigen Schicht (40) in Verbindung steht.

10. Absorbierender Artikel (10; 10a; 10b) nach einem der Ansprüche 1 bis 7, wobei die Deckschicht (34) aus drei Schichten aufgebaut ist, derart, daß eine Schicht aus einem Gemisch aus Zellstoff und polymerisiertem Alken sandwichartig zwischen zwei Trägerschichten aus polymerisiertem Alken angeordnet ist.

11. Absorbierender Artikel (10; 10a; 10b) nach einem der vorhergehenden Ansprüche, wobei das polymerisierte Alken Polyethylen, Polypropylen oder ein Gemisch aus Polyethylen und Polypropylen ist.

12. Absorbierender Artikel (10; 10a; 10b) nach einem der vorhergehenden Ansprüche, wobei die Deckschicht (34) ein Pigment enthält.

13. Absorbierender Artikel (10; 10a; 10b) nach Anspruch 12, wobei das Pigment Titandioxid ist.

14. Absorbierender Artikel (10; 10a; 10b) nach einem der vorhergehenden Ansprüche, wobei die Kompensationsschicht (32) ein Vliesmaterial enthält.

15. Absorbierender Artikel (10; 10a; 10b) nach Anspruch 14, wobei das Vliesmaterial aus polymerisiertem Alken und/oder Bikomponentenfasen aufgebaut ist.

16. Absorbierender Artikel (10; 10a; 10b) nach einem der vorhergehenden Ansprüche, wobei die Kompensationsschicht (32) auf der der Sekundärspeicherschicht (20) zugewandten Oberfläche mit einer oberflächenaktiven Substanz, welche vorzugsweise siliconhaltig ist, beschichtet ist.

17. Absorbierender Artikel (10; 10a; 10b) nach einem der vorhergehenden Ansprüche, wobei die Primärspeicherschicht (14) ein UCTAD-Material, Tissuewatte oder ein polymeres Alken enthält.

18. Absorbierender Artikel (10; 10a; 10b) nach einem der vorhergehenden Ansprüche, wobei die Primärspeicherschicht (14) aus einer Bahn aufgebaut ist, deren Randbereiche derart eingefaltet sind, daß diese sich gegenseitig überlappen.

19. Absorbierender Artikel (10; 10a; 10b) nach einem der vorhergehenden Ansprüche, wobei die flüssigkeitsundurchlässige Schicht (12) aus einem polymerisierten Alken in Folienform aufgebaut ist.

20. Absorbierender Artikel (10; 10a; 10b) nach einem der vorhergehenden Ansprüche, wobei die flüssigkeitsdurchlässige Schicht (40) aus einem polymerisierten Alken in Vliesform aufgebaut ist.

21. Absorbierender Artikel (10; 10a; 10b) nach Anspruch 17 oder 18, wobei das polymerisierte Alken Polyethylen, Polypropylen oder ein Gemisch daraus ist.

22. Absorbierender Artikel (10; 10a; 10b) nach einem der vorhergehenden Ansprüche, wobei die flüssigkeitsdurchlässige Schicht (40) eine zentrale Öffnung aufweist.

23. Absorbierender Artikel (10; 10a; 10b) nach einem der vorhergehenden Ansprüche, wobei an der flüssigkeitsundurchlässigen Schicht (12) mindestens ein Haftelement und/oder eine Haftschicht zur Befestigung des absorbierenden Artikels an einem Kleidungsstück angeordnet ist.

24. Absorbierender Artikel (10; 10a; 10b) nach einem der vorhergehenden Ansprüche, wobei seitlich an dem absorbierenden Artikel Flügel angeordnet sind.

25. Absorbierender Artikel (10; 10a, 10b) nach einem der vorhergehenden Ansprüche, wobei der absorbierende Artikel ein Hygieneprodukt, insbesondere eine Damenbinde oder Damen-Hygieneeinlage, ist.

## Claims

1. Absorbent article (10;10a;10b) having the following structure:
a liquid-impermeable bottom layer (12) disposed away from the wearer's body when said absorbent article is in use,
a primary storage layer (14) provided above said liquid-impermeable layer (12),
a secondary storage layer (20) provided above said primary storage layer (14),
a compensation layer (32) provided above said secondary storage layer (20),
a cover layer (34) with a central opening (36; 46) provided above said compensation layer (32), and
a liquid-permeable top layer (40) which is disposed toward the wearer's body when the absorbent article is in use,
**characterized in that** the cover layer (34) comprises a mixture of pulp and polymerized alkene, and wherein said mixture comprises at least 50% wt. % of polymerized alkene.

2. Absorbent article (10;10a;10b) according to claim 1, wherein said secondary storage layer (20) comprises at least one densified section.

3. Absorbent article (10;10a;10b) according to claim 2, wherein the at least one densified section extends in longitudinal direction of said article.

4. Absorbent article (10;10a;10b) according to claim 3, wherein the at least one densified section extending in longitudinal direction of said article is disposed at least within the projection of said central opening (36;46) of said cover layer (34) on said secondary storage layer (20).

5. Absorbent article (10;10a;10b) according to one of claims 2 to 4, wherein said secondary storage layer (20) comprises five densified sections.

6. Absorbent article (10;10a;10b) according to one of the preceding claims, wherein said secondary storage layer (20) is constructed of pulp.

7. Absorbent article (10;10a;10b) according to any of the preceding claims, wherein the amount of polymerized alkene is 50 to 80 wt.%, in particular 60 wt.%.

8. Absorbent article (10;10a;10b) according to any of the preceding claims, wherein the cover layer (34) is constructed of two layers such that a first layer of a mixture of pulp and polymerized alkene is applied on a second carrier layer of polymerized alkene, said first layer of a mixture of pulp and polymerized alkene communicating with said liquid-permeable layer (40) disposed towards the wearer's body when said absorbent article is in use and said second carrier layer communicating with said compensation layer (32).

9. Absorbent article (10;10a;10b) according to one of claims 1 to 7, wherein said cover layer (34) is constructed of two layers such that a first layer of a mixture of pulp and polymerized alkene is applied on a second carrier layer of polymerized alkene, said first layer of a mixture of pulp and polymerized alkene communicating with said compensation layer (32) and said second carrier layer communicating with said liquid-permeable layer (40) disposed toward the wearer's body when said absorbent article is in use.

10. Absorbent article (10;10a;10b) according to one of claims 1 to 7, wherein the cover layer (34) is constructed of three layers such that one layer of a mixture of pulp and polymerized alkene is sandwiched between two carrier layers of polymerized alkene.

11. Absorbent article (10;10a;10b) according to one of the preceding claims, wherein said polymerized alkene is polyethylene, polypropylene or a mixture of polyethylene and polypropylene.

12. Absorbent article (10;10a;10b) according to one of the preceding claims, wherein said cover layer (34) contains a pigment.

13. Absorbent article (10;10a;10b) according to claim 12, wherein said pigment is titanium dioxide.

14. Absorbent article (10;10a;10b) according to one of the preceding claims, wherein said compensation layer (32) includes a nonwoven material.

15. Absorbent article (10;10a;10b) according to claim 14, wherein said nonwoven material is constructed of polymerized alkene and/or bicomponent fibers.

16. Absorbent article (10;10a;10b) according to one of the preceding claims, wherein said compensation layer (32) is coated on the surface disposed toward said secondary storage layer (20) with a surface-active substance containing preferably silicone.

17. Absorbent article (10;10a;10b) according to one of the preceding claims, wherein said primary storage layer (14) includes an UCTAD material, tissue fluff or a polymer alkene.

18. Absorbent article (10;10a;10b) according to one of the preceding claims, wherein said primary storage layer (14) is constructed of a web, the peripheral portions of which are folded inwardly such that they overlap each other.

19. Absorbent article (10;10a;10b) according to one of the preceding claims, wherein said liquid-impermeable layer (12) is constructed of a polymerized alkene in the form of a film.

20. Absorbent article (10;10a;10b) according to one of the preceding claims, wherein said liquid-permeable layer (40) is constructed of a polymerized alkene in the form of a nonwoven.

21. Absorbent article (10;10a;10b) according to claim 17 or 18, wherein the polymerized alkene is polyethylene, polypropylene or a mixture thereof.

22. Absorbent article (10;10a;10b) according to one of the preceding claims, wherein said liquid-permeable layer (40) comprises a central opening.

23. Absorbent article (10;10a;10b) according to one of the preceding claims, wherein at least one adhesive element and/or adhesive layer for attachment of the absorbent article to a garment is provided on the liquid-impermeable layer (12).

24. Absorbent article (10;10a;10b) according to one of the preceding claims, wherein flaps are provided laterally at the absorbent article.

25. Absorbent article (10;10a;10b) according to one of the preceding claims, wherein said absorbent article is a sanitary product, in particular a sanitary napkin or feminine care liner.

## Revendications

1. Article absorbant (10 ; 10a ; 10b) présentant la structure suivante :
une couche inférieure imperméable aux fluides (12) orientée à l'opposé du corps lors de l'utilisation de l'article absorbant,
une couche de stockage primaire (14) placée au-dessus de la couche imperméable aux fluides(12) ;
une couche de stockage secondaire (20) placée au-dessus de la couche de stockage primaire (14),
une couche de compensation (32) placée au-dessus de la couche de stockage secondaire (20),
une couche de recouvrement (34) placée au-dessus de la couche de compensation (32) et présentant une ouverture centrale (36 ; 46), et
une couche supérieure perméable aux fluides (40), orientée vers le corps lors de l'utilisation de l'article absorbant,
**caractérisé en ce que** la couche de recouvrement (34) contient un mélange de cellulose et d'alcène polymérisé, ce mélange contenant au moins 50 % en poids d'alcène polymérisé.

2. Article absorbant (10 ; 10a ; 10b) selon la revendication 1 dont la couche de stockage secondaire (20) comporte au moins une zone densifiée.

3. Article absorbant (10 ; 10a ; 10b) selon la revendication 2 dont au moins une première zone densifiée est située dans le sens de la longueur de l'article.

4. Article absorbant (10 ; 10a ; 10b) selon la revendication 3 dont au moins la première zone densifiée située dans le sens de la longueur de l'article est placée sur la couche de stockage secondaire (20), au moins dans la projection de l'ouverture centrale (36 ; 46) de la couche de recouvrement (34).

5. Article absorbant (10 ; 10a ; 10b) selon l'une des revendications 2 à 4 dont la couche de stockage secondaire (20) comporte cinq zones densifiées.

6. Article absorbant (10 ; 10a ; 10b) selon l'une des revendications précédentes dont la couche de stockage secondaire (20) est constituée de cellulose.

7. Article absorbant (10 ; 10a ; 10b) selon l'une des revendications précédentes dont le pourcentage en poids d'alcène polymérisé est de 50 à 80 %, et plus particulièrement de 60 %.

8. Article absorbant (10 ; 10a ; 10b) selon l'une des revendications précédentes dont la couche de recouvrement (34) est constituée de deux couches, de sorte qu'une première couche faite d'un mélange de cellulose et d'alcène polymérisé est placée sur une deuxième couche de support faite d'alcène polymérisé, la première couche faite d'un mélange de cellulose et d'alcène polymérisé communiquant avec la couche perméable aux fluides (40) tournée vers le corps lors de l'utilisation de l'article absorbant et la deuxième couche de support communiquant avec la couche de compensation (32).

9. Article absorbant (10 ; 10a ; 10b) selon l'une des revendications 1 à 7 dont la couche de recouvrement (34) est constituée de deux couches, de sorte qu'une première couche faite d'un mélange de cellulose et d'alcène polymérisé est placée sur une deuxième couche de support faite d'alcène polymérisé, la première couche faite d'un mélange de cellulose et d'alcène polymérisé communiquant avec la couche de compensation (32) et la deuxième couche de support communiquant avec la couche perméable aux fluides (40) tournée vers le corps lors de l'utilisation de l'article absorbant.

10. Article absorbant (10 ; 10a ; 10b) selon l'une des revendications 1 à 7 dont la couche de recouvrement (34) est constituée de trois couches, de sorte qu'une première couche, faite d'un mélange de cellulose et d'alcène polymérisé, est intercalée entre deux couches de support faites d'alcène polymérisé.

11. Article absorbant (10 ; 10a ; 10b) selon l'une des revendications précédentes dans lequel l'alcène polymérisé est du polyéthylène, du polypropylène ou un mélange de polyéthylène et de polypropylène.

12. Article absorbant (10 ; 10a ; 10b) selon l'une des revendications précédentes dont la couche de recouvrement (34) contient un pigment.

13. Article absorbant (10 ; 10a ; 10b) selon la revendication 12 dont le pigment est du dioxyde de titane.

14. Article absorbant (10 ; 10a ; 10b) selon l'une des revendications précédentes dont la couche de compensation (32) contient un matériau non tissé.

15. Article absorbant (10 ; 10a ; 10b) selon la revendication 14 dans lequel le matériau non tissé est fait d'alcène polymérisé et/ou de fibres biconstituées.

16. Article absorbant (10 ; 10a ; 10b) selon l'une des revendications précédentes dont la couche de compensation (32) est revêtue, sur sa surface tournée vers la couche de stockage secondaire (20), d'une substance tensioactive contenant de préférence du silicone.

17. Article absorbant (10 ; 10a ; 10b) selon l'une des revendications précédentes dont la couche de stockage primaire (14) contient un matériau UCTAD (matériau non crêpé séché par soufflage transversal), un tissu ouaté ou un alcène polymère.

18. Article absorbant (10 ; 10a ; 10b) selon l'une des revendications précédentes dont la couche de stockage primaire (14) est constituée d'une bande dont les bords sont repliés de façon à se chevaucher.

19. Article absorbant (10 ; 10a ; 10b) selon l'une des revendications précédentes dont la couche imperméable aux fluides(12) est constituée d'un alcène polymérisé de structure feuilletée.

20. Article absorbant (10 ; 10a ; 10b) selon l'une des revendications précédentes dont la couche perméable aux fluides (40) est constituée d'un alcène polymérisé de structure non tissée.

21. Article absorbant (10 ; 10a ; 10b) selon la revendication 17 ou 18 dans lequel l'alcène polymérisé est du polyéthylène, du polypropylène ou un mélange de ces deux substances.

22. Article absorbant (10 ; 10a ; 10b) selon l'une des revendications précédentes dont la couche perméable aux fluides (40) présente une ouverture centrale.

23. Article absorbant (10 ; 10a ; 10b) selon l'une des revendications précédentes dans lequel au moins un élément adhésif et/ou une couche adhésive, destiné(e) à fixer l'article absorbant sur un vêtement, est disposé(e) sur la couche imperméable aux fluides(12).

24. Article absorbant (10 ; 10a ; 10b) selon l'une des revendications précédentes sur les côtés duquel sont placées des ailettes.

25. Article absorbant (10 ; 10a ; 10b) selon l'une des revendications précédentes constituant un produit d'hygiène, en particulier une serviette ou une protection hygiénique.
